# EUROPEAN PATENT SPECIFICATION

(11) **EP 2 550 366 B1**
(45) Date of publication and mention of the grant of the patent: **12.11.2014**
(21) Application number: 11710752.4
(22) Date of filing: 22.03.2011
(51) Int. Cl.: C12Q 1/18

(54) **Methods for identifying inhibitors of the type iii secretion system**
Verfahren zur Erkennung von Hemmern des Sekretionssystems des Typs III
Procédés d'identification des inhibiteurs du système de sécrétion de type III

(30) Priority: 23.03.2010 EP 10157397
(43) Date of publication of application: 30.01.2013
(73) Proprietor: IMBA-Institut für Molekulare Biotechnologie GmbH, 1030 Wien (AT)
(72) Inventor: MARLOVITS, Thomas, C., 1170 Wien (AT); RADICS, Julia, 1070 Wien (AT); SCHMIED, Wolfgang, 1020 Wien (AT)
(74) Representative: Sonn & Partner Patentanwälte
(86) International application number: PCT/EP2011/054379
(87) International publication number: WO 2011/117259

(56) References cited:
- WO-A2-2009/137133
- VEENENDAAL ANDREAS K J ET AL: "Small-Molecule Type III Secretion System Inhibitors Block Assembly of the Shigella Type III Secreton", JOURNAL OF BACTERIOLOGY, vol. 191, no. 2, January 2009 (2009-01), pages 563-570, XP002585349, ISSN: 0021-9193
- SANI MUSA ET AL: "IpaD is localized at the tip of the Shigella flexneri type III secretion apparatus", BIOCHIMICA ET BIOPHYSICA ACTA - GENERAL SUBJECTS, ELSEVIER SCIENCE PUBLISHERS, NL LNKD- DOI:10.1016/J.BBAGEN.2006.10.007, vol. 1770, no. 2, 1 February 2007 (2007-02-01), pages 307-311, XP002479751, ISSN: 0304-4165 [retrieved on 2006-10-18]

## Description

The present invention relates to methods for identifying anti-bacterial agents that target the type III secretion system (T3SS).

Although antibiotics are among the most effective drugs, they are associated with serious problems, e.g. their lack of specificity for the pathogenic bacteria, thereby affecting the normal bacterial flora, which results in the emergence of resistant bacteria. In search for alternative anti-bacterial drugs, the T3SS has evolved as a promising therapeutic target in the development of new therapeutics, because it is a key virulence mechanism that is highly conserved and absolutely required for virulence of many Gram-negative bacterial pathogens, which makes T3SS an attractive target.

Type III secretion systems are molecular machines that are fundamental for the virulence of important bacterial pathogens in that they deliver bacterial toxins ("effectors" or "effector proteins") into host cells, including *Salmonella enterica*, *Salmonella typhimurium, Salmonella typhi, Salmonella enteritica*, all other *Salmonella* species, *Shigella* spp (including *S. flexneri* and *S. dysenteriae*), *Yersinia* spp. (*Yersinia pestis, Yersinia pseudotuberculosis, Yersinia enterocolitica*), enteropathogenic strains of *E. coli* (EPEC*)*, enterohemorrhagic *E. coli* (EHEC), *Vibrio cholerae, Hafnia alvei, Bordetella* sp. and *Chlamydia* species. In addition, the conserved secretion pathway is needed for disease in animal pathogens such as rabbit *E. coli* (RDEC-1), and *Citrobacter rodentiii.* It is also critical for disease production in several important plant pathogens. Several Gram-negative pathogens cause economically important damage to plant crops using the c type III secretion system, as do human pathogens. Plant pathogens include *Pseudomonas syringe, P. solanacearum,* and *Xanthamonas campestris.*

The secreted effectors have the capacity to modulate various functions of the eukaryotic cell, which may be a mammalian cell or a plant cell, including cytoskeleton dynamics, vesicle traffic, cell cycle progression and transcription. Infection results in clinical symptoms ranging from mild headaches and diarrhea to life-threatening diseases such as typhoid fever or bubonic plague. Central to the T3SS is the needle complex, which is embedded within the inner and outer bacterial membrane, spans the periplasmic space, and extends into the extracellular environment with a needle-like filament. The cylindrically shaped needle complex ("injectisome") is composed of structural proteins which form a multi-ring base associated to the bacterial envelope, and a needle-like extension that protrudes several nanometers from the bacterial surface. The needle is anchored to the base through another substructure, the inner rod, which together with the needle filament forms a channel that serves as conduit for the effector proteins that travel this secretion pathway (Marlovits et al., 2004, Science 306, 1040-1042). Assembly of the needle complex occurs in discrete steps that first lead to the assembly of the base substructure.

The assembly and operation of the injectisome involves small (12-18 kDa) chaperones that remain in the bacterial cytosol (see e.g. Ghosh, 2004, Microbiol Mol Biol Rev 68: 771-795). While some of these chaperones are involved in the assembly of the injectisome (class III) or the translocation pore (class II), others are ancillary to effectors. The latter group of chaperones is classified as class I chaperones (Letzelter et al., 2006, The EMBO Journal (2006) 25, 3223-3233) and also designated "class 1A chaperones". These chaperones commonly bind to one effector, and most of them are encoded by genes located adjacent to the gene encoding the cognate effectors. They are acidic (pI: 4-5), usually dimeric, proteins, which bind their cognate effector within their first 100 amino acids, just downstream of the short N-terminal secretion signal. They are often, but not always, encoded next to the gene encoding their partner effector protein. Although their sequence similarity is low, their structure is quite well conserved. Before their translocation into host cells, chaperones are removed from effectors by the ATPase that is part of the injectisome (Akeda and Galan, 2005; Nature 437: 911-915).

The methods reported for identifying inhibitors of the T3SS mostly focus on secretion of the effector proteins and rely on reporter systems: WO2009/145829 describes a high throughput screening method based on a recombinant reporter protein (β-lactamase), the secretion of which is dependent on the type III protein secretion system, wherein a change in the amount of reporter protein that is secreted outside the recombinant bacterial host cell is indicative of an inhibiting or activating effect of a test compound on the T3SS.

WO2009/137133 describes a method that comprises, as a primary screening step, a similar reporter assay, in which the compound is tested for inhibiting secretion, which primary screen is followed by secondary assays in order to eliminate unspecific inhibitors. In the secondary assay, the compound is tested for transcriptional regulation of a T3SS protein and/or expression of a structural component of the T3SS.

The screening method for identifying compounds that affect type III secretion as described in WO2009/061491 involves assaying for inhibitor molecules that affect the so-called "low calcium response" in bacteria that have a type III secretion system. The primary screening assay is based on measuring bacterial growth, optionally using a reporter system. A secondary confirmation assay measures the effect of a test compound on the level secreted proteins, i.e. the effector proteins, which are, in the case of *Yersinia*, known as YOPs (2Yersinia outer proteins2).

Gauthier et al., Antimicrobial Agents and Chemotherapy, Oct. 2005, 4101-4109, describe an ELISA-based high-throughput assay for monitoring the effect of test compounds on the effector protein EspB of Enteropathogenic *Escherichia coli* (EPEC), a human pathogen responsible for outbreaks of diarrhea.

Veenendal et al., J. bact. 191(2), 2009:563-570, relates to salicylidenacylhydrazides as potential inhibitors of the T3SS needle complex and methods to detect needle complexes of different lengths.

Sani et al., Biochem. Bioph. Acta 1770(2), 2007:307-311 describes density features at the tip of the needle complex due to cross-linking.

It was an object of the present invention to provide new methods for determining whether a test compound affects the function of the T3SS and thereby the virulence of a bacterium that is dependent from the T3SS. In particular, the novel methods should provide a highly sensitive assay for identifying compounds that are very specific by affecting the function of the needle complex, i.e. by interfering with the assembly of its structural components.

Thus, the present invention relates to a method for determining whether a test compound has the ability to inhibit the function of the type 3 secretion system T3SS, wherein a test compound is contacted with bacterial cells that have a T3SS and is tested,
a. in a first step, for its ability to inhibit secretion of an effector protein in said bacterial cells by determining the amount of a secreted effector protein due to its binding to its cognate chaperone molecule, wherein a reduced amount of effector protein bound to said chaperone as compared to the amount in a control sample from cells that have not been treated with said test compound is indicative for an inhibitory effect of said compound on secretion of said effector protein, and
b. in a second step, for its ability to inhibit the assembly of the structural components to form the T3SS needle complex.

In said first step, the amount of secreted effector or translocator ("translocators" or "translocator proteins" are proteins that help effectors to cross the membrane of eukaryotic cells) may be determined directly, e.g. in the laboratory scale, by electrophoresis followed by protein staining. The amount of type III secreted proteins in the supernatant can also be detected by an immunological method using antibodies directed to one or more of the secreted proteins. Suitable methods include, but are not limited to, Western Blot, Immunodot Blot and ELISA. Preferably, the assay of said first step is an ELISA. The antibody added to the immobilized effector may be either directly labeled, or it may be detected indirectly by addition, after washing off excess first antibody, of a molar excess of a second, labeled antibody directed against IgG of the animal species of the first antibody.

A conventional ELISA assay is usually conducted according to protocols well known in the art, for example as follows: The sample to be tested, e.g. the bacterial lysate, is contacted and incubated with the immobilized capture (or coat) reagent. Immobilization conventionally is accomplished by insolubilizing the capture reagents either before the assay procedure, e.g. by adsorption to a water-insoluble matrix or surface. The solid phase used for immobilization may be any inert support or carrier that is essentially water insoluble and useful in immunometric assays, including supports in the form of e.g. surfaces, particles, beads, porous matrices, etc. Examples of commonly used supports include small sheets, Sephadex, polyvinyl chloride, plastic beads, and assay plates or test tubes manufactured from polyethylene, polypropylene, polystyrene, and the like including 96-well microtiter plates, as well as particulate materials such as filter paper, agarose, cross-linked dextran, and other polysaccharides. Alternatively, reactive water-insoluble matrices such as cyanogen bromide-activated carbohydrates may be used and the reactive substrates are suitably employed for immobilization of the capture reagent. In a preferred embodiment, the immobilized capture reagents are coated on a microtiter plate, and in particular the preferred solid phase used is a multi-well microtiter plate that can be used to analyze several samples at one time.

The solid phase is coated with the capture reagent, which may be linked to the solid support by a non-covalent or covalent interaction or physical linkage. Techniques for attachment are well known in the art. If covalent, the plate or other solid phase is incubated with a cross-linking agent together with the capture reagent under conditions well known in the art such as for 1 hour at room temperature.

The coated plates are typically treated with a blocking agent that binds non-specifically to the binding sites and saturates them to prevent unwanted binding to the excess sites on the wells of the plate. Examples of appropriate blocking agents include, e.g., gelatine, bovine serum albumin, egg albumin, casein, and non-fat milk.

After coating and blocking, the sample is added to the immobilized phase. For sufficient sensitivity, the amount of sample added should be such that the immobilized capture reagents are in molar excess of the maximum molar concentration of free effector anticipated in the sample. The conditions for incubation of sample and immobilized capture reagent are selected to maximize sensitivity of the assay and to minimize dissociation. Next, the sample is removed (preferably by washing with a "washing buffer") to remove uncaptured effector.

A cross-linking agent or other suitable agent may also be added at this stage to allow the bound effector to be covalently attached to the capture reagents, in the case there were any concern that the captured effector may dissociate to some extent in the subsequent steps.

Next, the immobilized effector is contacted with an antibody, which is either directly or indirectly detectable. The antibody may be polyclonal or monoclonal. The antibody may be directly detectable, e.g. carrying a fluorimetric label. The fluorimetric label has greater sensitivity compared to the colorimetric label. The detectable antibody may be biotinylated and the detection means may be avidin or streptavidin-beta-galactosidase and MUG (4-methylumbelliferyl-beta-galactoside).

Preferably, a molar excess of an antibody with respect to the maximum expected concentration of free effector is added to the plate after it has been washed. The affinity of the antibody must be sufficiently high that small amounts of the free effector can be detected.

Next, the amount of effector that is bound to the capture reagent is measured by detecting the effector-bound antibody. To this end, the antibody may be either directly labeled, or detected indirectly by addition, after washing off of excess first antibody, of a molar excess of a second, labeled antibody directed against IgG of the animal species of the first antibody. In the latter, indirect assay, labeled antisera against the first antibody are added to the sample so as to produce the labeled antibody in situ.

The label used for either the first or second antibody is any detectable functionality that does not interfere with the binding of the binding partners. Examples of suitable labels are those known in the art for use in immunoassays, including moieties that may be detected directly, such as a fluorochrome, chemiluminscent, and radioactive labels, as well as moieties, such as enzymes, that need to be reacted or derivatized for detection. Examples of such labels include the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g. firefly luciferase and bacterial luciferase, luciferin, 2,3-dihydrophthalazinediones, horseradish peroxidase (HRP), alkaline phosphatase, beta.-galactosidase, glucoamylase, lysozyme, saccharide oxidases, e.g. glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, biotin/streptavidin, biotin/Streptavidin-beta-galactosidase with MUG, spin labels, bacteriophage labels, stable free radicals, and the like.
Conventional methods are available to bind these labels covalently to proteins or polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels.

The conjugation of such label, including enzymatic labels, to the antibody is a standard procedure for one of ordinary skill in immunoassay techniques. See, for example, O'Sullivan et al. "Methods for the Preparation of Enzyme-antibody Conjugates for Use in Enzyme Immunoassay," in Methods in Enzymology, ed. J. J. Langone and H. Van Vunakis, Vol. 73 (Academic Press, New York, N.Y., 1981), pp. 147-166.

Following the addition of the labeled antibody, the amount of bound antibody is determined by removing excess unbound labeled antibody by washing and then measuring the amount of the attached label, using a detection method specific to the label, and correlating the measured amount with the amount of effector in the sample. For example, in the case of enzymes, the amount of color developed and measured may be correlated to the amount of effector. Specifically, if HRP is the label, the colour is detected using the substrate OPD and measuring at 490 nm absorbance.

In one example, after an enzyme-labeled second antibody directed against the first unlabeled antibody has been washed from the immobilized phase, color or chemiluminiscence is developed and measured by incubating the immobilized capture reagent with a substrate of the enzyme. Then the amount of secreted concentration is calculated by comparing with the color or chemiluminescence generated by the standard effector run in parallel.

ELISAs that are useful for the present invention have been described by Gauthier et al., Antimicrobial Agents and Chemotherapy, Oct. 2005, 4101-410, and in WO1999/45136 In brief, bacteria from an overnight culture are subcultured in microtiter plates to allow the secreted effector protein of interest to adhere to the plate (alternatively, the supernatant from centrifuged bacteria may be used). The plate-bound effector (or translocator) is detected with an anti-effector antibody or an anti-translocator antibody, respectively, and a peroxidase-conjugated secondary antibody. The test is developed by adding o-phenylenediamine dihydrochloride (OPD), and, after quenching, analyzed in a plate reader.

According to the invention in said first step, the amount of secreted effector protein is determined due to its ability to bind to (interact with) its cognate chaperone molecule. (For the purpose of the invention, in this context, the term "chaperone" is synonymous for class I chaperones (or class 1A chaperones) as defined above, i.e. chaperone molecules that are required for secretion of the effectors.) Thus, the assay of the first step is based on measuring the amount of effector that is bound to its chaperone.

The amount of secreted effector can be determined by any method that is suitable for assaying a protein that is bound to a binding partner.

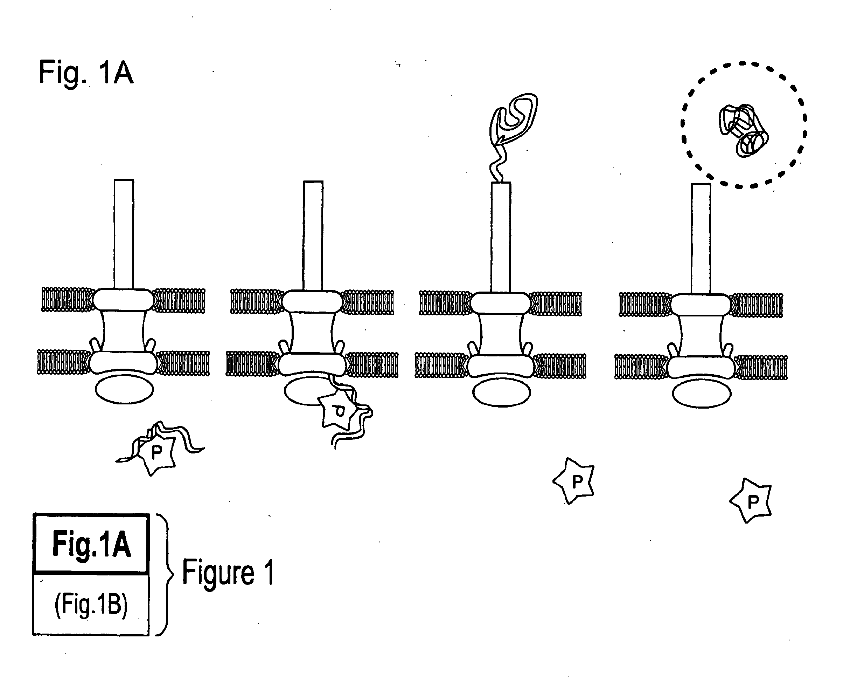

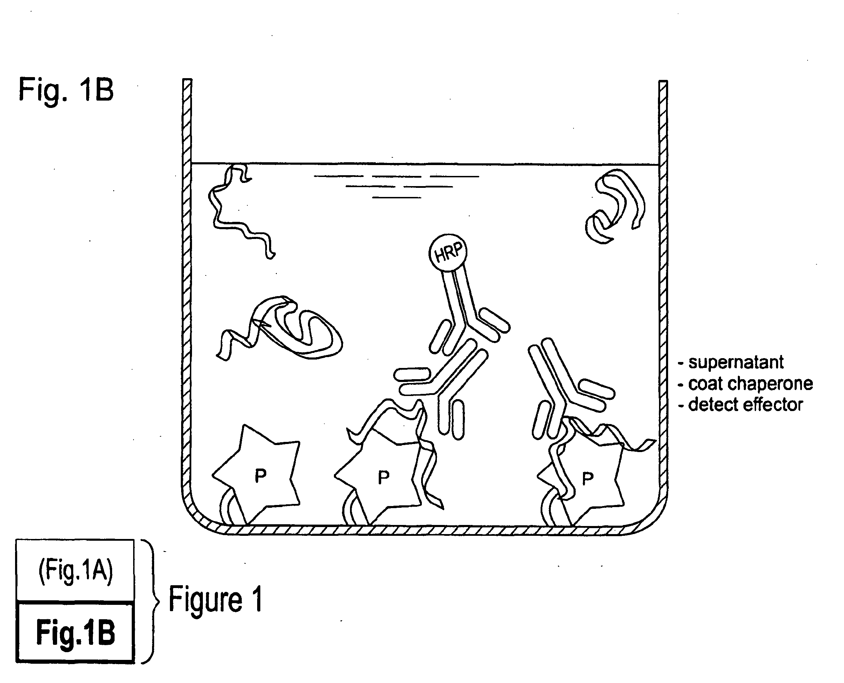

According to certain embodiments, the effector protein is detected by means of an ELISA-type assay which may be carried out according to the principle described above, using commercially available standard methodology. By way of example, such an assay may be conducted as follows: the chaperone is immobilized on a microtiter plate (e.g. by a plate-bound antibody against the chaperone or a fragment thereof or against a chaperone-bound tag, e.g. GST, His, Myc). The lysate or supernatant from a culture of bacteria that normally secrete the effector protein and that have been treated with the test compound (in parallel, a control sample from untreated bacteria), is added to the plate and the amount of effector protein determined by means of an antibody against the effector protein and a secondary antibody that carries a detectable label as described above, e.g. horseradish peroxidase, alkaline phosphatase or a fluorophor. Figure 1A schematically depicts the mechanism of secretion of an effector that is bound to a cognate chaperone (designated P in the figure) before being secreted. Figure 1B exemplifies an ELISA, in which chaperon P is coated onto a plate, and the effector is determined by measuring the amount of a labelled secondary antibody that binds to the primary anti-effector antibody.

Preferably, an ELISA is used as described in Example 1 (for the purpose of this invention, such ELISA is termed "secretion ELISA"; in the case that another than the ELISA format is used, such assay is termed "secretion assay"). A secretion ELISA allows detection of secreted molecules, e.g. effector molecules, directly from culture supernant. The primary advantage is the possibility to test up to 96 samples per multiwell plate, including the measurement of both culture growth and effector within 24 hours. It has been successfully used so far to test 384 samples per man-day, and is routinely used instead of TCA (trichloroacetic acid) precipitation and western blotting. By way of example, a secretion assay may be established using the effector/chaperone pairs SptP/SicP (as described in Example 1), or SipA-Flag/InvB.

While being exemplified by *Salmonella typhimurium,* the above described principle may be applied to any other pathogenic Gram-negative bacteria that secrete the effector protein by means of the T3SS. Thus, the assay may be based on any combination of proteins that represent a chaperone/effector pair, using, as the coated component, the respective chaperone for the respective effector that is to be detected. The effect of the compound can be analyzed by comparing the measured amount with that of a control where the bacterial cell has not been treated with the compound.

Examples for effector proteins from bacteria other than *Salmonella typhimurium* are YopE, YopH, YpkA/YopO, YopP/YopJ, YopM, YopT, from *Yersinia* spp.; Tir from *E. coli*; ExoS, ExoT, ExoY from *P. aeruginosa*; IpaA, IpaB, and IpgD from *Shigella* spp.; AvrPto *from P. syringae pv. Tomato*; AvrBs2 from *Xanthomonas campestris pv. Vesicatoria*; and AvrBs3 from *X. campestris pv. Vesicatoria.*
E.g. for identifying an inhibitor of *Yersinia,* instead of effector SptP (from *Salmonella*), chaperone SycT may be used as the coated protein and the effector to be detected is YopT. The chaperons and effector proteins (including their protein and DNA sequences) as well combinations are known from the literature, examples of useful effectors and effector/chaperone pairs are given in Tables 1 - 5. Table 1: *Salmonella enterica*; Table 2: *Yersinia pestis* / *Yersinia enterocolitica l Yersinia pseudotuberculosis;* Table 3: *Escherichia coli*; Table 4: *Shigella flexneri* / *Shigella sonnei*; Table 5: *Chlamydia (Chlamydia pneumoniae* / *Chlamydia trachomatis* / *Chlamydia muridarum* / *Chlamydophila felis*).

In the case that a chaperon for a given effector has not (yet) been identified, its existence may be determined, e.g. using bioinformatics analyses as described by Pallen et al., 2005; BMC Microbiol. 5: 9.

Often, full activation of a type III protein secretion system requires that the bacterial cell contacts its host cell. Under *in vitro* conditions, expression of components of a T3SS may be low. Therefore, as described in WO2005/113791 preferably, the method of the invention further comprises the step of activating the type III protein secretion system prior to the step of detecting the amount of effector secreted outside the bacterial cell. The step of activating the type III protein secretion system can be performed either before or after exposing the host cell to a test compound. Methods are known to those skilled in the art to increase the activity of a type III protein secretion system in a given bacterial cell, and are described in WO2005/113791, e.g. by directly contacting a bacterial cell with its eukaryotic host cell or by applying selected *in vitro* laboratory conditions such as temperature, osmolarity, nutrients, divalent cations like Ca²⁺, pH value, and growth phase. For example, to trigger Yop secretion *in vitro, Yersinia* is generally grown at 28°C in a medium depleted of Ca²⁺ and then transferred to 37°C. As in *Yersinia* spp., also in *P. aeruginosa* secretion of proteins is activated under low-Ca²⁺ conditions. Growing *Shigella* or enteropathogenic *E. coli* at 37°C activates their T3SS. For *Salmonella typhimurium,* the SPI-I type III protein secretion system preferably is activated *in vitro* under low-oxygen, high-osmolarity, and slightly alkaline (pH 8) conditions.

In a preferred embodiment, the first step of the method of the invention is a screening method carried out in the high-throughput format, for which several useful assays are commercially available.

According to preferred embodiments, the high-throughput assay relies on the bead-based Alpha technology ("Amplified Luminescent Proximity Homogeneous Assay"; PerkinElmer), which is suitable for conversion of ELISA assays (so-called "AlphaLISAs") and allows highly sensitive, homogeneous assays that are easily automated.

This technology is based on a signal that depends on the proximity of a so-called donor bead and an acceptor bead. AlphaScreen relies on the use of "Donor" and "Acceptor" beads that are coated with a layer of hydrogel providing functional groups for bioconjugation. The Donor beads are further coated with a photosensitizer layer which upon excitation with 680 nm generates singlet oxygen. If the beads are brought into close proximity, the half-life of singlet oxygen is sufficiently long to travel to the Acceptor bead and elicit chemiluminescence.

By way of example, when the effector is detected directly, i.e. without being bound to its chaperone, the assay is in the so-called sandwich format, which requires two antibodies which recognize different epitopes on the effector. One antibody is biotinylated and is captured by the streptavidin-coated donor bead. The second antibody is coupled to the acceptor bead. The presence of the effector results in an immunosandwich, thus bringing the donor and acceptor beads into close proximity. If the concentration of effector is high, donor-acceptor immunosandwiches are formed, resulting in an increased signal.

In the case that the assay is carried out according to the embodiment that is based on the interaction of the effector with its cognate chaperon, by way of example, one bead carries the chaperon and the other bead an anti-effector antibody. The chaperon may be directly conjugated to the beads or, alternatively, streptavidin-coated standard beads may be used to which the biotinylated chaperon molecules are bound.

Detection may be direct or indirect: in the direct assay format, the anti-effector antibody or the chaperone is conjugated directly onto the bead. This provides the convenience of a ready-to-screen assay. In an indirect assay, a secondary antibody, or Protein A, is conjugated to the bead. This method minimizes the use of primary antibody when it is either very expensive or difficult to obtain.

By way of example, the assay may be performed as a two-step reaction by incubating, e.g. for 1 h, the sample with beads coated with biotinylated chaperone and beads coated with anti-effector antibody. This is followed by incubation with beads covalently coated with streptavidin for 30 min. After the incubation steps, light generated from a chemiluminescent reaction within the beads is quantitated. The assay may be run in 384-well plates with a sample volume of 5 µL.

Other, albeit less preferred, assay types for the high-throughput format are selected from fluorescence detection technologies, e.g. time resolved fluorescence assays like the dissociation-enhanced lanthanide fluoroimmunoassay (DELFIA) or fluorescence resonance energy transfer (FRET). DELFIA uses a lanthanide chelate with a long fluorescent lifetime (>100 µs) to avoid background interference from assay components such as buffers, media, reagents or compounds. The label absorbs light in the UV range from a nitrogen laser or flash lamp and, depending on the lanthanide used, emits fluorescence between 500 and 700 nm. This assay includes wash steps, but this drawback may, in some instances, be accepted as compensation for the benefit of high detection.

The majority of commercial DELFIA kits are based on non-competitive "sandwich type" assays. By way of example, for the present invention, the chaperone molecule is bound to the solid support, while an anti-effector antibody is labeled, e.g. with Europium. The amount of measured Eu directly correlates with the amount of effector bound to the chaperon.

In a further aspect, the present invention relates to a method for monitoring the assembly of the structural components to form the T3SS needle complex, wherein assembly of the needle complex is determined by detecting a later-attached structural component [x] when it is associated with a pre-existing structural component [x-1], or with a pre-formed complex that contains component [x-1], respectively.

In the method of the invention for determining whether a test compound has the ability to inhibit the function of the T3SS, this method represents the second step.

In said second step (also termed, for the purpose of this invention "structure assay" or "structure ELISA" when in the ELISA format), a positive hit from the first step, i.e. a compound that inhibits secretion of an effector or a translocator, is tested for its ability to inhibit the assembly of the structural components to form the needle complex. This assay step makes use of the fact that assembly is a stepwise process, in which a structural component [x] is associated with a pre-existing structural component [x-1], or with a pre-formed complex that contains component [x-1], respectively.

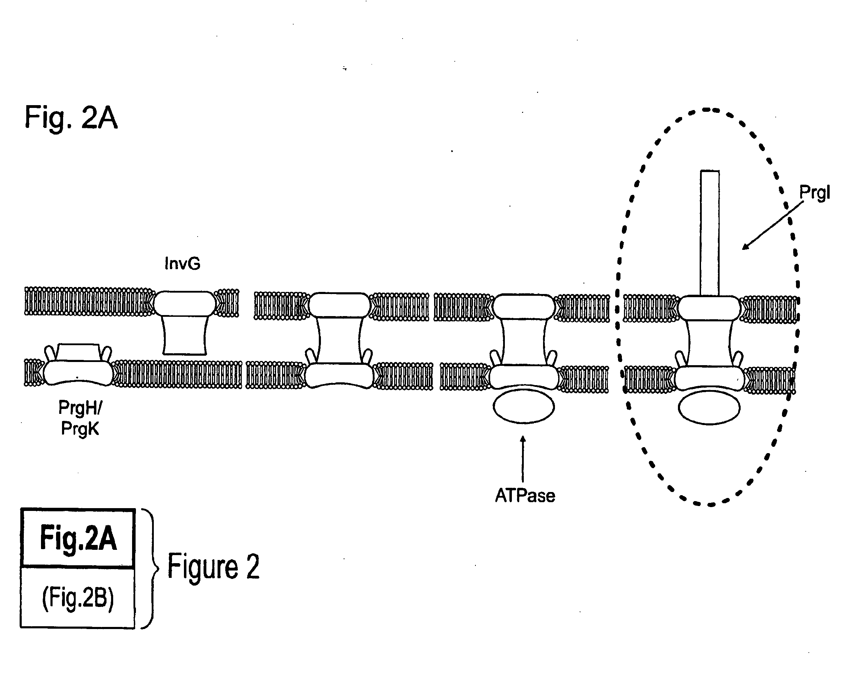

Exemplified by *Salmonella typhimurium,* the base substructure is made up by the structural components PrgH, PrgK and InvG, while PrgI and PrgJ constitute the needle and inner rod substructures, which, in the chain of the naturally occurring assembly, are added after the base has been formed. Based on this sequence, by way of example, PrgI may be component [x], while PrgH represents [x-1]. Alternatively, PrgK or InvG may represent component[x-1], or a complex consisting PrgH and PrgK or a complex containing all three base components PrgH, PrgK and InvG. Exemplified by these two structural components, the assay is designed such that PrgH is captured on test plates and PrgI, if associated with the pre-formed base that contains PrgH, is detected. Figure 2A schematically depicts the assembly of the injectisome.

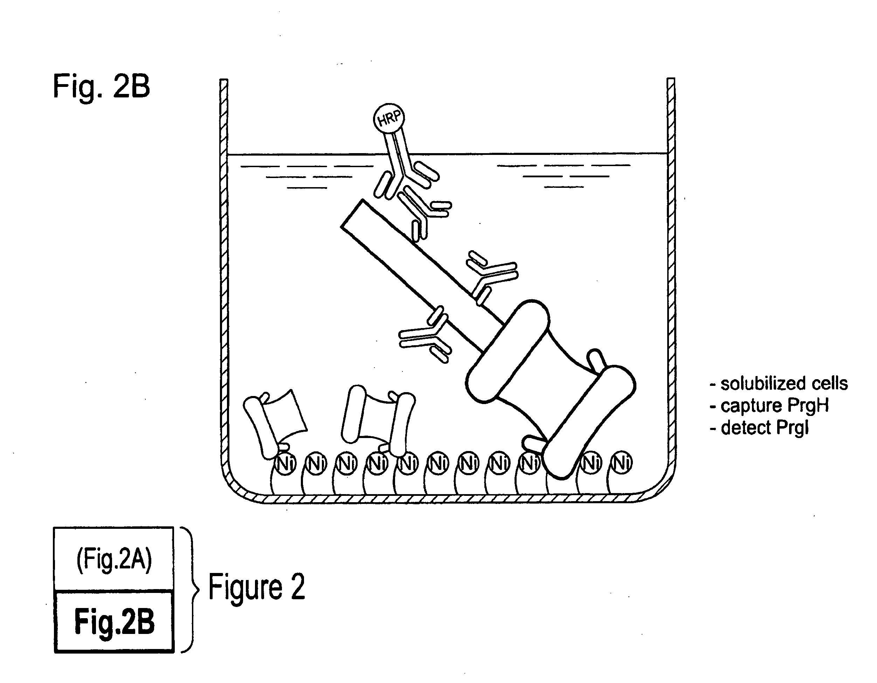

Figure 2B exemplifies an ELISA, in which PrgH is bound to a plate, and the attachment of the needle filament component PrgI is determined by measuring the amount of a labelled secondary antibody that binds to the primary anti-PrgI antibody.

To carry out the structure ELISA assay of said second step, by way of example, *Salmonella typhimurium* cells carrying a plasmid that encodes a tagged PrgH, e.g. a His-tagged PrgH, are grown, in the presence or absence of said test compound. The cells are lysed and the lysate is added to Nickel-coated test plates such that His-tagged PrgH contained in the bacterial lysate will bind to the test plates. After a period of time sufficient for PrgH to bind to the plates, the plates are washed. In the next step, the plates are assayed for the presence of PrgI: In the absence of the test compound (or in the presence of a test compound that does not affect the association of PrgI with the pre-formed base structure), PrgI is bound to the pre-formed complex and can be detected by means of a primary anti-PrgI antibody and a secondary antibody carrying a detectable label. Alternatively to expressing structural component [x-1] as a His-tag fusion protein, it may carry another tag, e.g. the GST tag (human glutathione S-transferase), which is bound to a plate coated via GST antibodies. Other commonly used fusion tags are the HA-tag, MBP, Myc-tag.

In the case that the structure assay does not reveal an inhibitory effect on the assembly of PrgI with the pre-formed base complex, other combinations of structural component [x-1] / [x] from Salmonella are tested, e.g. the PrgH/PrgK complex as component [x-1] and InvG as component [x].

A negative result for a test compound with regard to an inhibitory effect on the attachment of PrgI may be due to a test compound's inhibitory effect on the ATPase, which is associated with the base of the T3SS and is required for directing proteins into the needle. Such inhibitory effect on the ATPse will be confirmed and may be further analysed, e.g. to find out whether the compound inhibits the catalytic function or whether targets another part of the enzyme. The results of such analyses are useful as a starting point for optimizing a compound as an inhibitor specific for the T3SS-associated ATPase of that bacterium.

While being exemplified by *Salmonella typhimurium,* the above described principle of the structure ELISA may be applied to any other pathogenic Gram-negative bacteria that use the T3SS. Thus, the structure assay may utilize any of the structural components that form the needle complex, using, component [x-1] as the coated component, and the later-assembled protein [x] as the component to be detected. By way of example, to identify an inhibitor of *Shigella flexneri,* instead of PgH, component mxiG may be used as the coated protein and instead of PrgI, mxiH is the component to be detected. The respective components are known from the literature, examples of structural proteins corresponding to PrgH, PrgI and InvG, that may be used in the structural assay of the invention, are given in Table 6. Homologs to other structural proteins are found in the literature.

The compounds identified to be inhibitors of the T3SS of a specific bacterium can be tested whether they also block the T3SS of another Gram-negative bacterium. Usually, the compound is first tested for its ability to inhibit secretion of an effector or translocator of the other bacterium according to the methods described above. If the result is positive, it may be expected that a compound that has been shown to interfere with a certain assembly step in a first bacterium, may be expected to inhibit the same assembly step of the second bacterium. Therefore, the structure assay for the second bacterium will comprise the structural components that are homologous to those of the first bacterium. To obtain specificity of a compound as an inhibitor of the second bacterium, the compound will be modeled into relevant structural components of either of the two bacteria.

The method of the invention allows for identifying inhibitors of the T3SS that are highly specific in that they interfere with a discrete step in the assembly of the injectisome. Once a compound has been shown to interfere with the attachment of a structural component [x] to a component [x-1], crystal structures of these components may be obtained, and the structure of a test compound may be optimized by modeling into or on such structure.

The compounds identified by the method of the invention to be inhibitors of the T3SS are further tested in animal disease models by toxicity testing. Compounds that block T3SS can be tested in relevant animal disease models, e.g. EPEC/EHEC in rabbits, and *S. typhimurium* in mice. Compounds are typically first tested for their toxicity to mice using standard methods known to those of skill in the art. Initially the pharmacokinetics of the drug is tested by measuring the levels of the drug in the blood within hours following oral drug ingestion. This information provides an indication of the bioavailability of the compound. Toxicity testing is also performed to determine the maximum tolerated dose. To do this, increasing concentrations of the drug are administered to an animal up to a level of 1000 mg/kg.

Initial testing in animal disease models may be performed e.g. by inhibition of EPEC- mediated disease. Once initial toxicity and bioavailability studies have been completed, animal infection models are tested with the most promising compounds. These standard assays are used to determine the effect of promising compounds on RDEC-1 (the rabbit EPEC pathogen) and RDEC-1 containing the verotoxin (a well-known EHEC animal model) in rabbits. The amount of diarrhea is measured, and pathology is performed on infected animals to determine the extent of colonization with and without the drug. These studies indicate whether type III secretion compounds can affect the outcome of these infections.

Compounds that have been shown to have an effect on the T3SS of *Salmonella typhimurium* by means of the method of the invention, are tested, as described in WO 1999/045136 in animal disease models by determining inhibition of *Salmonella typhimurium* disease in the murine typhoid model. *S. typhimurium* infection in Balb/C mice leads to murine typhoid, ultimately resulting in animal death. Two routes of infection are used: oral (LD₅ₒ=10⁶) and intravenous (LD₅o=10²). Instead, animals can be sacrificed at various times, their livers and spleens homogenized, and the number of *S. typhimurium* in these organs counted according to the method of Leung et al. (Proc. Nat. Acad. Sci. USA. 88: 11470-4, 1991). This technique is an indicator of infectiveness. To test the ability of inhibitors to block *S. typhimurium* virulence, the mice are given various doses of the compound at the same time as oral and IV infection. The dose depends on the results of the toxicity and bioavailability tests. The ability of these compounds to alter organ colonization rates is an excellent indicator of the effectiveness of these compounds as potential antibacterial therapeutics.

### Brief description of the Figures:

Figure 1:
   A) Mechanism of secretion of an effector that is bound to a cognate chaperone
   B) Secretion ELISA for detection of an effector bound to its cognate chaperone
Figure 2:
   A) Mechanism of the assembly of the injectisome
   B) Structure ELISA for detection of an assembled needle complex

### Example 1

### Secretion ELISA for detecting the effector protein SptP when bound to its cognate chaperone SicP

GST-SicP is expressed in *E. coli* (BL21, plasmid X), the cells lysed and the protein purified by high pressure liquid chromatography (HPLC) on a GST-trap column. The final protein concentration of SicP for precoating is 60 µg/ml in TBS. 96 well flat bottom multiwell plates (300 µl, Microtest™ 96-well ELISA plates clear, BD Falcon, US) are washed with 100 µl TBS per well and precoated with 100 µl of SicP in solution for either 2 hours at room temperature, or at 4°C over night while slowly shaking. All liquid is removed and the plates stored at -80°C. ELISAs are performed with plates stored for up to a year.
Bacteria are grown in 2 ml deepwell plates (Riplate® sw 2 ml, Ritter) in 1.8 ml LB growth medium (0,3 M NaCl, 0,012% arabinose, antibiotics) for at least 16 hours. Culture supernatant is obtained by pelleting cells at 4000 g for 15 minutes (Multifuge 3 SR, Heraeus, UK).

Precoated plates are wrapped in paper to avoid condensation and warmed to room temperature. During incubation steps, the plate is covered with aluminium foil and placed on a shaker, the protocol is best performed at room temperature. A washing step consists of adding and removing 200 µl of PBS-T per well.
Unspecific binding is minimized by blocking with 300 µl 3% bovine serum albumin (BSA) in PBS for one hour, after which the blocking solution is removed without a washing step. 100 µl of culture supernatant are loaded for one hour to capture the effector protein, followed by three washing steps. 100 µl of the first antibody dilution (1:3000 monoclonal anti-SptP from mouse in PBS-T) are added for one hour, followed by three washing steps. 100 µl of the second antibody dilution (1:15000 α-mouse from rabbit, coupled to horse radish peroxidase (HRP)) are added for one hour, followed by three washing steps. For the final detection step, 100 µl of freshly prepared substrate are applied. 3,3',5,5'-tetramethylbenzidin (TMB) is prepared as a 100x stock solution by dissolving 10 mg/ml in dimethylsulfoxide (DMSO). The working solution is prepared by mixing 100 µl of 100x stock solution, 2 µl of 30% H₂O₂ and 10 ml 0.1 M citrate buffer (pH 6), and is at room temperature when applied.
While a blue color is developing, the reaction kinetic is measured using a plate reader (GENios Pro, Tecan, US) at 640 nm. As soon as a distinct blue color is displayed, the reaction is stopped using 25 µl of 2 M sulfuric acid (H₂SO₄) and the absorbance is measured at 450 nm.

The above described assay may be converted to the HTS format, e.g. an AlphaScreen, and/or employ, for identifying inhibitors of other Gram-negative bacteria, effectors and effector/chaperone pairs from such bacteria. Examples are given in Tables 1 - 5.

**Table 1**

| ***Salmonella enterica*** | | | | |
|---|---|---|---|---|
| **Effector** | **AccNr** | | **Chaperone** | **AccNr** |
| SptP | P74873 | | SicP | O85300 |
| SopB | 030916 | | SigE | O30917 |
| SopA | B5QZK6 | | InvB | P0A1N0 |
| SopE2 | Q7CQD4 | | InvB | P0A1N0 |
| SipA | Q56027 | | InvB | P0A1N0 |
| SopE | O52623 | | InvB | P0A1N0 |
| SipB | Q56019 | | SicA | P69066 |
| SipC | Q56020 | | SicA | P69066 |
| | | | | |
| SseF | 084951 | | SscA | O84946 |
| SseB | Q7BVH7 | | SpiC | P74863 |
| SseC | 084947 | | SpiC | P74863 |
| SseL | Q8ZNG2 | | SrcA | Q8ZNP3 |
| PipB2 | D0ZTZ7 | | SrcA | Q8ZNP3 |
| | | | | |
| AvrA | 030621 | | --- | |
| CopR | Q8ZQ53 | | --- | |
| SteA | Q8ZPD7 | | --- | |
| SteB | Q8ZPA6 | | --- | |
| SteC | Q8ZP57 | | --- | |
| PipB | Q8ZQ59 | | --- | |
| SifA | Q56061 | | --- | |
| SifB | Q9KIB9 | | --- | |
| SlrP | Q8ZQQ2 | | --- | |
| SsaH | Q9ZEF4 | | --- | |
| SsaI | Q9ZEF3 | | --- | |
| SsaJ | P74852 | | --- | |
| SseG | O84952 | | --- | |
| SseI | Q8ZQ79 | | --- | |
| SseJ | Q9FD10 | | --- | |
| SspH1 | D0ZVG2 | | --- | |
| SspH2 | D0ZPH9 | | --- | |
| SteA | Q8ZPD7 | | --- | |
| SteB | D0ZI38 | | --- | |
| SteC | Q8ZP57 | | --- | |
| SipD | Q56026 | | --- | |
| SseD | Q9R803 | | --- | |
| SpvB | C0Q8H0 | | --- | |

**Table 2**

| ***Yersinia*** | | | | |
|---|---|---|---|---|
| **Effector** | **AccNr** | | **Chaperone** | **AccNr** |
| YopN | P68640 | | SycN/YscB | P61380/Q56973 |
| YopT | O68703 | | SycT | P0C2V9 |
| YopE | P31493 | | SycE | Q79NJ9 |
| YscX | P0C2N4 | | YscY | P61417 |
| YopB | Q06114 | | SycD | C5IZG5 |
| YopD | Q06131 | | SycD | C5IZG5 |
| YopH | P08538 | | SycH | Q56934 |
| YopO | Q93KQ6 | | SycO | Q84GR4 |
| | | | | |
| YopTl | P0C2N1 | | --- | |
| YpkA | Q05608 | | --- | |
| LcrV | Q3I759 | | --- | |
| YopP | O52162 | | --- | |
| YopM | P17778 | | --- | |
| YopJ | A1JUC5 | | --- | |
| VirG | A1JU90 | | --- | |
| YsaH | A1JQC0 | | --- | |
| YspB | A1JQ86 | | --- | |
| YsaW | A1JQA7 | | --- | |
| TyeA | A9R9I1 | | --- | |
| YopR | D1U2F5 | | --- | |
| YsrR | Q9KKI8 | | --- | |
| YitR | Q8CLV0 | | --- | |
| YitA | Q8D1P8 | | --- | |
| YitB | Q8D1P7 | | --- | |
| YitC | Q8D1P6 | | --- | |
| YipA | Q7CL72 | | --- | |
| YipB | Q8CLU9 | | --- | |

**Table 3**

| ***Escherichia coli*** | | | | |
|---|---|---|---|---|
| **Effector** | **AccNr** | | **Chaperone** | **AccNr** |
| SepD | Q5WME1 | | --- | |
| Map | B8ZYG0 | | CesT | Q47015 |
| SepZ | B8ZYN7 | | CesT | Q47015 |
| Tir | Q9KWH9 | | CesT | Q47015 |
| EspD | Q7DB81 | | CesD2 | O52150 |
| EspF | Q7DB85 | | CesF | C6UYM0 |
| EspB | Q8XC86 | | CesAB | O52124 |
| EspD | Q7DB81 | | CesD | Q9AJ22 |
| EspA | Q7DB80 | | CesAB | O52124 |
| EspB | Q8XC86 | | CesD | Q9AJ22 |
| NleA | A9ZNG8 | | CesT | Q47015 |
| NleH | A9ZNE5 | | CesT | Q47015 |
| NleF | C6USQ4 | | CesT | Q47015 |
| --- | | | Rorf8 | C8UFL9 |
| --- | | | YgeG | C8UAJ9 |
| EspH | B8ZYG2 | | --- | |
| EspJ | C6UYI2 | | --- | |
| NleA1 | A1KWP2 | | --- | |
| NleA2 | A1KWP3 | | --- | |
| NleA3 | A1KWP4 | | --- | |
| NleA4 | A1KWP5 | | --- | |
| NleA5 | A1KWP6 | | --- | |
| NleA6-1 | A1KWP7 | | --- | |
| NleA6-2 | A1KWP8 | | --- | |
| NleA7 | A1KWP9 | | --- | |
| NleA8-1 | A1KWQ0 | | --- | |
| NleA8-2 | A1KWQ1 | | --- | |
| NleA9 | A1KWQ2 | | --- | |
| NleA10 | A1KWQ3 | | --- | |
| NleA11 | A1KWQ4 | | --- | |

**Table 4**

| ***Shigella*** | | | | |
|---|---|---|---|---|
| **Effector** | **AccNr** | | **Chaperone** | **AccNr** |
| IpaA | P18010 | | SpaK | P35530 |
| IpgB1 | Q6XVY7 | | SpaK | P35530 |
| OspC3 | Q3YTS0 | | SpaK | P35530 |
| OspB | Q3YTY8 | | SpaK | P35530 |
| IpaB | P18011 | | IpgC | P0A2U4 |
| IpaC | P18012 | | IpgC | P0A2U4 |
| IcsB | P33546 | | IpgA | P33547 |
| IpgD | Q07566 | | IpgE | Q6XVY3 |
| OspF | Q8VSP9 | | --- | |
| VirA | Q3YTK0 | | --- | |
| OspC1 | Q3YTU3 | | --- | |
| OspC2 | Q3YTS7 | | --- | |
| OspC3 | Q3YTS0 | | --- | |
| OspD1 | Q3YTX0 | | --- | |
| OspD2 | Q3YTY4 | | --- | |
| OspD3 | Q3YTU2 | | --- | |
| OspE1 | Q9AJU4 | | --- | |
| OspE2 | Q9AJW6 | | --- | |
| IpaH3 | Q83RJ4 | | --- | |
| OspG | Q3YTH2 | | --- | |
| OspE1 | Q327E9 | | --- | |
| OspE2 | Q3YTU8 | | --- | |
| IpaH9.8 | O85159 | | --- | |
| IpaH4.5 | P18009 | | --- | |
| IpaH7.8 | P18014 | | --- | |

**Table 5**

| **Chlamydia** | | | | |
|---|---|---|---|---|
| **Effector** | **AccNr** | | **Chaperone** | **AccNr** |
| CopB | O84582 | | Scc2 (SycD) | A9NIN9 |
| CopB2 | O84583 | | Scc3 (SycD) | Q9PJG4 |
| CT694 | O84700 | | --- | |
| pkn5 | O84680 | | --- | |
| Tarp | Q6GX35 | | --- | |
| IncA | O69196 | | --- | |
| IncB | Q9Z8P7 | | --- | |
| IncC | Q3KMC9 | | --- | |
| CopN | Q9Z8L4 | | --- | |
| CPn0206 | Q9Z8X8 | | --- | |
| CPn0330 | Q9Z8K8 | | --- | |
| CPn0374 | Q9Z8G9 | | --- | |
| CPn0474 | Q9Z877 | | --- | |
| CPn0490 | Q9Z861 | | --- | |
| CPn0648 | Q9Z7Q6 | | --- | |
| CPn0671 | Q9Z7N3 | | --- | |
| CPn0705 | Q9Z7K0 | | --- | |
| CPn0725 | Q9Z7I0 | | --- | |
| CPn0761 | Q9Z7E5 | | --- | |
| CPn0764 | Q9Z7E2 | | --- | |
| CPn0770 | Q9Z7D6 | | --- | |
| CPn0774 | Q9Z7D2 | | --- | |
| CPn0808 | Q9Z798 | | --- | |
| CPn0809 | Q9Z797 | | --- | |
| CPn0821 | Q9Z785 | | --- | |
| CPn0853 | Q9Z753 | | --- | |
| CPn0859 | Q9Z747 | | --- | |
| CPn0879 | Q9Z727 | | --- | |
| CPn1005 | Q9Z6Q4 | | --- | |
| CPn1019 | Q9Z6P0 | | --- | |
| CPn1020 | Q9Z6N9 | | --- | |
| CPn1022 | Q9Z6N7 | | --- | |
| CPn1032 | Q9Z6M7 | | --- | |
| --- | | | Scc1 (SycE1) | 034021 |
| --- | | | SycE2 | |
| --- | | | SycE3 | |
| --- | | | lcrH-2 | Q9Z6N8 |

### Example 2

### Structure ELISA to detect the attachment of PrgI to the PgH-containing base element

The method used in this experiment allows for monitoring a fully assembled needle complex with a high throughput rate. It is based on capturing the needle complex (PrgH-poly-histidine tag) on Ni-NTA plates and detecting the needle filament by using anti-PrgI antibody. Thereby, only a fully assembled needle complex is detected.

The nonflagellated *Salmonella typhimurium* strain SB906 carrying the transcriptional regulator hilA under the araBAD promoter is complemented by a plasmid carrying PrgH with a C-terminal poly-histidine tag (His₁₈). Bacteria are grown in 2 ml deepwell plates (Riplate® sw 2 ml, Ritter) in 1.8 ml LB (0.3 M NaCl, 0,012% arabinose, antibiotics) for at least 18 hours. Cells are harvested by pelleting at 4000 g for 15 minutes (Multifuge 3 SR, Heraeus, UK). The cell pellet is resuspended in 400 µl lysis buffer (0.5 M Sucrose in 0.15 M Tris buffer, 0.48 mg/ml lysozyme, 5 mM EDTA) and incubated at 14°C for 45 minutes in a thermoshaker. The tubes are then incubated at 37°C for 20 minutes at 37°C. This step may be done either in a thermoshaker or in a waterbath, depending on the number of samples. 100 µl of LDAO/salt mixture (6 parts 10% LDAO, 44 parts 5 M NaCl) are added to each sample, followed by another 5 minute incubation step at 37°C. 4 µl of MgCl₂ are added, followed by 5 minutes incubation time. The finished cell lysate is then kept at 4°C for 10-20 minutes.

20 µl of the cell lysate are diluted with 80 µl resuspension buffer (PBS, 0.5 M NaCl, 3% BSA, 0.5% LDAO, pH 8.0). The resulting solution is loaded onto pre-wetted (resuspension buffer) multiwell plates with nickel-coating (Ni-NTA Hisorb plates, Qiagen, USA), incubated for 1 hour, followed by three washing steps. A washing step includes adding 200 µl resuspension buffer, slowly shaking the plate for 5 minutes and removing the solution. For this protocol, all incubations are performed at room temperature on a slowly shaking plate, covered by aluminum foil.

Antibodies are diluted in resuspension buffer and left on the multiwell plate for 1 hour. Anti-PrgI (1:2000; anti-PrgI antibody raised in rabbits against recombinant PrgI-his6) is added first and removed by three washing steps after one hour incubation time. Anti--rabbit peroxidase-coupled antibodies (1:10000) are used as secondary antibodies, again incubated for one hour and washed off by three washing steps. The final detection is performed by adding 100 µl of Tetramethylbenzidine supersensitive (Sigma, US). As soon as a distinct blue color is displayed, the reaction is stopped using 20 µl of 2 M sulfuric acid and the absorbance is measured at 450 nm.

Examples of PrgH and PrgI homologs that may be used to identify inhibitors of the assembly of other Gram-negative bacteria are listed in Table 6.

**Table 6**

| **Accession** | **Entry name** | **Protein names** | **Gene names** | **Organism** |
|---|---|---|---|---|
| | | | | |

| **PrgH homologs** | | | | |
|---|---|---|---|---|
| B2VEF8 | B2VEF8_ ERWT9 | Type III secretion system protein | prgH (ETA_19100) | Erwinia tasmaniensis (strain DSM 17950 / Et1/99) |
| Q7DB78 | Q7DB78_ ECO57 | EscD (Type III secretion system protein EscD) (Type III secretion system EscD protein) | escD (ECs4558) (Z5109) | Escherichia coli 0157:H7 |
| P41783 | PRGH_ SALTY | Protein prgH | prgH (STM2874) | Salmonella typhimurium |
| P0A221 | MXIG_SHIFL | Protein mxiG | mxiG (CP0136) | Shigella flexneri |
| Q6R8E0 | Q6R8E0_ SODGL | YsaF | ysaF | Sodalis glossinidius |
| A9R9J7 | A9R9J7_ YERPG | Type III secretion apparatus protein YscD | yscD (YpAngola_ B0055) | Yersinia pestis bv. Antiqua (strain Angola) |
| | | | | |

| **PrgI homologs** | | | | |
|---|---|---|---|---|
| B2VEF9 | B2VEF9_ ERWT9 | Type III secretion apparatus | prgI (ETA_19110) | Erwinia tasmaniensis (strain DSM 17950 / Etl/99) |
| B7N793 | B7N793_ ECOLU | Type III secretion system needle protein | prgI (ECUMN_ 3190) | Escherichia coli O17:K52:H18 (strain UMN026 / ExPEC) |
| P41784 | PRGI SALTY | Protein prgI | prgI (STM2873) | Salmonella typhimurium |
| P0A223 | MXIH_SHIFL | Protein mxiH | mxiH (CP0137) | Shigella flexneri |
| Q6R8A5 | Q6R8A5_ SODGL | PrgI | prgI | Sodalis glossinidius |
| Q01247 | YSCF_ YEREN | Yop proteins translocation protein F | yscF | Yersinia enterocolitica |
| | | | | |

| **InvG homologs** | | | | |
|---|---|---|---|---|
| B2VEF5 | B2VEF5_ ERWT9 | Type III secretion apparatus | invG (ETA 19080) | Erwinia tasmaniensis (strain DSM 17950 / Etl/99) |
| B7UMB3 | B7UMB3_ ECO27 | T3SS structure protein EscC | escC (E2348_C_ 3955) | Escherichia coli O127:H6 (strain E2348/69 / EPEC) |
| P35672 | INVG_ SALTY | Protein invG | invG (STM2898) | Salmonella typhimurium |
| Q04641 | MXID_SHIFL | Outer membrane protein mxiD | mxiD (CP0145) | Shigella flexneri |
| Q6R8B7 | Q6R8B7_ SODGL | InvG | invG | Sodalis glossinidius |
| Q7BRZ9 | Q7BRZ9_ YEREN | Secretin YscC | yscC | Yersinia enterocolitica |

### Example 3

### Immunodetection of the translocators SipB and SipC (Western Blot)

A *Salmonella typhimurium* culture is grown under conditions promoting needle complex formation, with an uninduced preculture over night, followed by 5 to 10 hours of induced growth, or with direct inoculation and growth over night in liquid LB (0.3 M NaCl, 0,012% arabinose, suitable antibiotics), both at 37°C.

The culture is cleared of cells by centrifugation in tubes. The supernatant of the main culture is passed through a 0.45 µm filter. 1.5 ml of trichloroacetic acid (TCA, 100% w/v stock solution) are added to 10 ml of protein sample and stored at 4°C for at least 60 min. For the following steps, centrifuges are cooled to below 0°C. Acetone used for washing the sample is stored at 18°C and kept on ice on the bench.

The precipitate is pelleted by centrifugation in falcon tubes at 8000 rpm, for 40 min, the supernatant is carefully discarded and the pellet washed with 1.8 ml cold acetone. The sample is then transferred to 2 ml plastic tubes and is stored at 20°C. Proteins are pelleted in a refrigerated table top centrifuge at maximum speed (13 krpm, 10 min), the supernatant removed and 1.8 ml of cold acetone (-20°C) added. The last step is repeated, the supernatant taken off and the pellet dried on a heating block set to 95°C for a few minutes. The pellet is resuspended in 40 µl H2O and 60 µl 5x Lammli sample buffer. The sample is heated to 98°C for 5 min on a heating block before loading on a polyacrylamide gel.

A polyacrylamide gel with separated protein samples is washed by placing it in a plastic container with 100 ml of H2O and gently shaken for 5 minutes at room temperature. The H2O is then replaced by protein transfer buffer (PTB: 2.9 g glycine, 5.8 g Tris base, 0.37 g sodium dodecyl sulfate, 200 ml methanol, in 800 ml dH2O), and left submerged for another 10 minutes.

A PVDF membrane (Millipore, Billerica, USA) and a suitable amount of filter paper (2x three layers of Whatman® 3MM Chr filter paper) are cut to size. The membrane is activated by wetting with 100% methanol, then washed for 5 minutes in dH2O, followed by 10 minutes equilibration in 1x PTB. The filter paper is wetted using 1x PTB. The transfer of proteins from the gel to the membrane is done by using Bio-Rads Trans-Blot SD Semi-Dry Transfer Cell. After blotting, the membrane is carefully placed in a suitable plastic container, in which all incubation steps take place while slowly shaking.

Additional binding sites are blocked by soaking the membrane in 5% non-fat dried milk dissolved in 0.1% Tween 20 in PBS (PBS-T) for 1h at room temperature or over night at 4°C. The membrane is rinsed twice with PBS-T and then placed in a dilution of the primary detection antibody in PBS-T (anti-SipB 1:10000, raised in rabbits against recombinant SipB, anti-SipC 1:1000 raised in rabbits against recombinant SipC). The membrane is incubated for one hour at room temperature, or over night at 4°C. Two rinsing steps and three washing steps, lasting each 5 minutes in PBS-T, are done before a dilution of the secondary antibody (antimouse 1:10000 in PBS-T) is added for 1 to 2 hours at room temperature. The membrane is again rinsed twice and washed three times before the detection reagents (Amersham™ ECL Western Blotting Detection Reagents; GE Healthcare, USA) are applied. Excessive liquid is carefully removed and the 1 ml of detection reagent pipetted and spread over each membrane. After one minute of incubation the membrane is placed in transparent film, placed in an x-ray cassette and the protein band could be detected by film exposure (Amersham™ Hyperfilm™, GE Healthcare, USA) for 10 seconds to 30 minutes. The exposed film is developed using a Colenta MP 900 F film developer. Identification of single protein is done by mass spectroscopy, both SipB and SipC are detected. This shows that the T3SS is functional under the chosen culture conditions. If the culture is grown, under the same conditions, in the presence of an inhibitor of the functionality of the T3SS, SipB and/or SipC are not detected.

In an analagous manner, effector molecules can be detected that do not have a chaperone.

Instead of using a Western Blot, for the HTS format, a sandwich-tpye AlphaLISA is carried out in the following way: One anti-SipB antibody (or anti-SipC antibody, respectively), is biotinylated and captured by a streptavidin-coated donor bead. A second anti-SipB antibody (or anti-SipC antibody, respectively) is coupled to the acceptor bead. The presence of SipB (or SipC respectively) results in an immunosandwich, thus bringing the donor and acceptor beads into close proximity, which results in a measurable signal.

## Claims

1. A method for determining whether a test compound has the ability to inhibit the function of the type 3 secretion system T3SS, wherein a test compound is contacted with bacterial cells that have a T3SS and is tested,
a. in a first step, for its ability to inhibit secretion of an effector protein in said bacterial cells by determining the amount of a secreted effector protein due to its binding to its cognate chaperone molecule, wherein a reduced amount of effector protein bound to said chaperone as compared to the amount in a control sample from cells that have not been treated with said test compound is indicative for an inhibitory effect of said compound on secretion of said effector protein, and
b. in a second step, for its ability to inhibit the assembly of the structural components to form the T3SS needle complex.

2. The method of claim 1, wherein the effector is selected from SptP and SipA-Flag and the chaperone is selected from SicP and InvB, respectively.

3. The method of claims 1 or 2, wherein the amount of secreted effector or trans locator protein is determined by an ELISA.

4. The method of claim 3, wherein the ELISA is converted to an Amplified Luminescent Proximity Homogeneous Assay and run in the high throughput format.

5. A method for monitoring the assembly of the structural components to form the T3SS needle complex, wherein the assembly of the needle complex is determined by detecting a later-attached structural component [x] when it is associated with a pre-existing structural component [x-1], or with a pre-formed complex that contains component [x-1], respectively.

6. The method of claim 1, wherein in said second step, assembly of the needle complex is determined by detecting a later-attached structural component [x] when it is associated with a pre-existing structural component [x-1], or with a pre-formed complex that contains component [x-1], respectively.

7. The method of claim 5 or 6, wherein the attachment of component [x-1] to component [x] is determined in an ELISA, wherein component [x-1] or a complex containing it is conjugated onto a solid support and component [x] is detected when attached to [x-1].

8. The method of any one of claims 5 to 7, wherein PrgI from *Salmonella typhimurium* or a homolog thereof from another Gram-negative bacterium is component [x] and wherein PrgH from *Salmonella typhimurium* or a homo log thereof from another Gram-negative bacterium is component [x-1].

## Patentansprüche

1. Verfahren zum Feststellen, ob eine Testverbindung die Fähigkeit besitzt, die Funktion des Typ-3-Sekretionssystems T3SS zu hemmen, wobei eine Testverbindung mit bakteriellen Zellen in Berührung gebracht wird, welche ein T3SS aufweisen und,
a. in einem ersten Schritt auf ihre Fähigkeit getestet wird, die Sekretion eines Effektorproteins in den bakteriellen Zellen zu hemmen, indem die Menge eines sekretierten Effektorproteins aufgrund seiner Bindung an sein entsprechendes Chaperonmolekül bestimmt wird, wobei eine reduzierte Menge des an das Chaperon gebundenen Effektorproteins im Vergleich zu der Menge in einer Kontrollprobe von Zellen, die nicht mit der Testverbindung behandelt worden sind, auf einen hemmenden Effekt der Verbindung auf die Sekretion des Effektorproteins hindeutet, und
b. in einem zweiten Schritt auf ihre Fähigkeit zum Hemmen der Assemblierung der strukturellen Komponenten zur Bildung des T3SS-Nadelkomplexes getestet wird.

2. Verfahren nach Anspruch 1, wobei der Effektor aus SptP und SipA-Flag ausgewählt ist und das Chaperon aus SicP bzw. InvB ausgewählt ist.

3. Verfahren nach Anspruch 1 oder 2, wobei die Menge an sekretiertem Effektor- oder Translokatorprotein mit einem ELISA festgestellt wird.

4. Verfahren nach Anspruch 3, wobei der ELISA zu einem ALPH-Assay (Amplified Luminescent Proximity Homogeneous Assay) umgewandelt wird und in einem Format mit hohem Durchsatz durchgeführt wird.

5. Verfahren zum Überwachen der Assemblierung der strukturellen Komponenten zur Bildung des T3SS-Nadelkomplexes, wobei die Assemblierung des Nadelkomplexes festgestellt wird, indem eine später angebrachte strukturelle Komponente [x] detektiert wird, wenn sie an eine bereits vorhandene strukturelle Komponente [x-1] oder an einen vorgeformten Komplex, der Komponente [x-1] enthält, angelagert ist.

6. Verfahren nach Anspruch 1, wobei in dem zweiten Schritt die Assemblierung des Nadelkomplexes festgestellt wird, indem eine später angebrachte strukturelle Komponente [x] detektiert wird, wenn sie an eine bereits vorhandene strukturelle Komponente [x-1] oder an einen vorgeformten Komplex, der Komponente [x-1] enthält, angelagert ist.

7. Verfahren nach Anspruch 5 oder 6, wobei die Anlagerung von Komponente [x-1] an Komponente [x] in einem ELISA festgestellt wird, wobei Komponente [x-1] oder ein Komplex, der sie enthält, auf einen festen Träger konjugiert ist und Komponente [x] detektiert wird, wenn sie an [x-1] angelagert ist.

8. Verfahren nach einem der Ansprüche 5 bis 7, wobei es sich bei Komponente [x] um PrgI von *Salmonella typhimurium* oder ein Homolog davon von einem anderen gramnegativen Bakterium handelt und es sich bei Komponente [x-1] um PrgH von *Salmonella typhimurium* oder ein Homolog davon von einem anderen gramnegativen Bakterium handelt.

## Revendications

1. Procédé pour déterminer si un composé de test possède une aptitude à inhiber la fonction du système de sécrétion de type 3 SST3, dans lequel un composé de test est mis en contact avec des cellules bactériennes qui possèdent un SST3 puis est testé,
a. lors d'une première étape, pour son aptitude à inhiber la sécrétion d'une protéine effectrice dans lesdites cellules bactériennes en déterminant la quantité de protéine effectrice sécrétée suite à sa liaison avec sa molécule chaperonne apparentée, où une quantité réduite de protéine effectrice liée à ladite chaperonne par comparaison à la quantité observée dans un échantillon de contrôle provenant de cellules qui n'ont pas été traitées avec ledit composé de test indique un effet inhibiteur dudit composé sur la sécrétion de ladite protéine effectrice, et
b. lors d'une seconde étape, pour son aptitude à inhiber l'assemblage des composants structurels qui forment le complexe d'aiguille du SST3.

2. Procédé selon la revendication 1, dans lequel l'effecteur est sélectionné parmi SptP et SipA-Flag et la chaperonne est sélectionnée parmi SicP et InvB, respectivement.

3. Procédé selon les revendications 1 ou 2, dans lequel la quantité de protéine effectrice ou de translocation sécrétée est déterminée par un ELISA.

4. Procédé selon la revendication 3, dans lequel l'ELISA est converti en un essai homogène de la luminescence de proximité amplifiée puis est exécuté dans un format à haut débit.

5. Procédé pour surveiller l'assemblage des composants structurels qui forment le complexe d'aiguille du SST3, dans lequel l'assemblage du complexe d'aiguille est déterminé en détectant un composant structurel attaché ultérieurement [x] lorsqu'il est associé à un composant structurel préexistant [x-1], ou à un complexe préformé qui contient le composant [x-1], respectivement.

6. Procédé selon la revendication 1, dans lequel lors de ladite seconde étape, l'assemblage du complexe d'aiguille est déterminé en détectant un composant structurel attaché ultérieurement [x] lorsqu'il est associé à un composant structurel préexistant [x-1], ou à un complexe préformé qui contient le composant [x-1], respectivement.

7. Procédé selon la revendication 5 ou 6, dans lequel l'attachement du composant [x-1] au composant [x] est déterminé dans un ELISA, le composant [x-1] ou un complexe le contenant étant conjugué sur un support solide et le composant [x] étant détecté lorsqu'il est attaché à [x-1].

8. Procédé selon l'une quelconque des revendications 5 à 7, dans lequel Prgl de *Salmonella typhimurium* ou un homologue de celle-ci issu d'une autre bactérie à Gram négatif est le composant [x] et dans lequel PrgH de *Salmonella typhimurium* ou un homologue de celle-ci issu d'une autre bactérie à Gram négatif est le composant [x-1].
